**Europäisches Patentamt**

**European Patent Office**

**Office européen des brevets**

(19)

(11) Veröffentlichungsnummer: **0 133 976**
A2

(12) # EUROPÄISCHE PATENTANMELDUNG

(21) Anmeldenummer: **84108963.4**

(22) Anmeldetag: **28.07.84**

(51) Int. Cl.⁴: **G 01 N 33/545**, G 01 N 33/577

(30) Priorität: **09.08.83 CH 4338/83**
**08.05.84 CH 2248/84**

(43) Veröffentlichungstag der Anmeldung: **13.03.85**
**Patentblatt 85/11**

(84) Benannte Vertragsstaaten: **AT BE CH DE FR GB IT LI NL**

(71) Anmelder: **F. HOFFMANN-LA ROCHE & CO.**
**Aktiengesellschaft, CH-4002 Basel (CH)**

(72) Erfinder: **Brodbeck, Hans, Lindenstrasse 12,**
**CH-4142 Münchenstein (CH)**
Erfinder: **Gallati, Harald, Dr., Ingelsteinweg 13,**
**CH-4143 Dornach (CH)**

(74) Vertreter: **Kloter, Rudolf et al,**
**Postfach 3255 Grenzacherstrasse 124, CH-4002 Basel**
**(CH)**

(54) **Reagens für Immunverfahren.**

(57) Die Erfindung betrifft ein Reagens zur Durchführung von Immunverfahren. Dieses Reagens enthält einen mit einem immunologisch aktiven Material beschichteten festen Träger, z.B. eine Polystyrolkugel. Der beschichtete Träger ist mit einem Salz einer Zuckersäure überzogen. Als Zuckersäure kommt insbesondere Glucuronsäure oder Gluconsäure in Betracht, und als Salz insbesondere das Natriumsalz.

F.Hoffmann-La Roche & Co.Aktiengesellschaft, Basel/Schweiz

0133976

RAN 4093/68

Reagens für Immunverfahren

Die vorliegende Erfindung betrifft ein Reagens zur Durchführung von Immunverfahren, enthaltend einen mit einem immunologisch aktiven Material beschichteten festen Träger.

Verschiedene immunologische Verfahren, insbesondere enzymimmunologische Verfahren, zur quantitativen oder qualitativen Bestimmung von diagnostisch und therapeutisch wichtigen Substanzen basieren auf dem Festphasen-Prinzip, bei dem ein wasserunlöslicher Träger verwendet wird, der mit einem immunologisch aktiven Material beschichtet ist.

Als wasserunlösliche Träger für solche Zwecke sind geeignet: organische und anorganische Polymere, z.B. Amylase, Dextrane, native oder modifizierte Cellulose, Polyacrylamid, Agarose, Magnetit, poröses Glaspulver, Polyvinylidenfluorid (Kynar), die Innenwand von Testgefässen (Teströhrchen, Titrierplatten oder Küvetten aus Glas oder Kunststoff) sowie die Oberfläche von festen Körpern (Glas- und Kunststoffstab, Stab mit endständiger Verdickung, Stab mit endständigen Flügeln oder Lamellen). Besonders geeignete Träger für die immunologischen Materialien sind oberflächenbehandelte Polystyrolkugeln, die in der Regel einen Durchmesser von ungefähr 6,35-6,5 mm aufweisen.

Klt/ 20.6.1984

Bisher mussten diese mit immunologischen Materialien beschichteten (sensibilisierten) Träger stets in Lösung aufbewahrt werden, da nach dem Trocknen der Träger eine wesentlich geringere immunologische Bindungskapazität gefunden wurde. Das Aufbewahren der Träger in Lösung bringt jedoch mancherlei Nachteile für die Praktikabilität dieser Tests mit sich.

In der deutschen Patentschrift Nr. 2910707 wird vorgeschlagen, die sensibilisierten Träger nach dem Waschen mit einem Puffer mit einer Zuckerlösung zu behandeln und dann während etwa 30 Minuten an der Luft zu trocknen. Die mit Zucker überzogenen sensibilisierten Träger zeigen auch nach längerer Lagerung keinen Verlust an immunologischer Aktivität. Andererseits zeigen diese Träger, insbesondere wenn Monosaccharide verwendet werden, die unangenehme Eigenschaft, dass sie im Kontakt mit Luftfeuchtigkeit klebrig werden, wodurch das manuelle und vor allem das maschinelle Arbeiten mit diesen Trägern erheblich erschwert wird.

Ueberraschenderweise wurde im Rahmen der vorliegenden Erfindung gefunden, dass der vorerwähnte Nachteil behoben werden kann, indem man die sensibilisierten Kugeln nicht mit Zucker sondern mit dem Salz einer Zuckersäure überzieht. Die auf diese Weise mit dem Salz einer Zuckersäure überzogenen sensibilisierten Kugeln zeigen den oben erwähnten Nachteil nicht, sind aber dennoch genau so gut lagerfähig wie die Träger gemäss der vorerwähnten deutschen Patentschrift Nr. 2910707.

Die vorliegende Erfindung betrifft demnach ein Reagens zur Durchführung von Immunverfahren, enthaltend einen mit einem immunologisch aktiven Material beschichteten festen Träger, welches dadurch gekennzeichnet ist, dass der beschichtete Träger mit einem Salz einer Zuckersäure überzogen ist.

Als Salz einer Zuckersäure kommen insbesondere ein Glucuronsäure-, Mannonsäure-, Galacturonsäure- sowie ein Gluconsäuresalz in Betracht. Als Salze eignen sich zweckmässig die Alkali-oder Ammoniumsalze. Besonders bevorzugt sind die Natriumsalze. Das bevorzugte Salz einer Zuckersäure ist das Natriumglucuronat oder das Natriumgluconat.

Als Immunverfahren kommt insbesondere ein Enzym-Immunverfahren in Betracht.

Das immunologisch aktive Material, mit dem der Träger beschichtet ist, ist insbesondere ein Antigen, ein Antikörper oder ein Hapten. Bevorzugte Antikörper sind Antikörper gegen das karzinoembryonale Antigen (CEA), gegen HCG, gegen Interferon, gegen Hepatitis B Oberflächenantigen sowie gegen humanes IgE. Besonders bevorzugt sind in diesem Zusammenhang monoklonale Mausantikörper, die in an sich bekannter Weise nach der Zellfusionstechnik nach Köhler und Milstein hergestellt werden. Beim Antikörper gegen Interferon kommt insbesondere ein Antikörper gegen rekombiniertes Interferon αA in Betracht.

Als zu beschichtender Träger kommt insbesondere eine oberflächenbehandelte Polystyrolkugel in Betracht, deren Durchmesser ca. 6,35-6,5 mm aufweist.

Zur Herstellung des Ueberzugs werden die in an sich bekannter Weise mit dem immunologisch aktiven Material beschichteten Träger einer Pufferlösung entnommen, abgetropft und dann in eine wässrige Lösung enthaltend das Salz einer Zuckersäure getaucht, deren pH im Bereich von 5-9 und insbesondere von 6-7,5 ist. Falls erforderlich, kann der pH der wässrigen Lösung mit einem Puffer eingestellt werden, wobei allerdings relativ wenig Puffer verwendet werden sollte, damit der beschichtete Träger nicht mit dem Puffer sondern mit dem Zuckersäuresalz überzogen wird.

Die wässrige Lösung enthält zwischen 5 und 200 g/l des Zuckersäuresalzes. Bevorzugt sind 20-100 g/l insbesondere 50 g/l.

Gegebenenfalls kann die Lösung einen Stabilisator, wie z.B. Thimerosal (Natrium-äthylmercurithiosalicylat) enthalten.

Nach Entfernen der Träger aus der Lösung mit dem Zuckersäuresalz werden diese getrocknet, wobei die Trocknungsart nicht kritisch ist. Bevorzugt erfolgt die Trocknung an der Luft zwischen einer Temperatur von 0-56°C, wobei eine Temperatur zwischen Raumtemperatur und 45°C bevorzugt ist. Besonders bevorzugt ist eine Temperatur von 37°C. Das Trocknen kann aber auch bei 37°C im Rotationsverdampfer am Vakuum oder mit dem Wirbelschichtgranulator erfolgen. Für grössere Chargen kann das Trocknen auch nach dem Wirbelschichtverfahren erfolgen.

Die Dauer der Trocknung ist ebenfalls nicht kritisch, doch hat sich gezeigt, dass sehr gute Resultate erhalten werden, wenn die Träger während 24 Stunden bei 37°C an der Luft getrocknet werden. Für das Trocknen unter Vakuum am Rotationsverdampfer genügt in der Regel ein 1- bis 2-stündiges Trocknen. Beim Trocknen mit dem Wirbelschichtgranulator genügt in der Regel ein ca. 10 minütiges Trocknen.

Die nachfolgenden Beispiele erläutern die Erfindung.

## Beispiel 1

Polystyrolkugeln (Durchmesser 6,35 mm) werden in Pufferlösung in herkömmlicher Weise mit monoklonalen Mausantikörpern gegen rekombiniertes Interferon αA sensibilisiert. Die Kugeln werden aus der Pufferlösung entnommen, in einem groben Sieb abgetropft und während 10 Minuten bei Raumtemperatur in einer wässrigen Lösung enthaltend 50 g/l Natriumglucuronat suspendiert. Anschliessend werden die Kugeln mit einem Sieb aus dieser Lösung entnommen, abgetropft und während 24 Stunden bei 37°C getrocknet.

Ein Teil der Kugeln wird bei Raumtemperatur während 1 Monat gelagert während ein anderer Teil während 1 Monat bei 37°C gelagert wird.

Alle Kugeln zeigen nach der Lagerung nur äusserst geringfügige Verluste an immunologischer Aktivität. Die Kugeln bleiben nach Kontakt mit Luftfeuchtigkeit trocken.

## Beispiel 1 a

Polystyrolkugeln (Durchmesser 6,35 mm) werden in Pufferlösung in herkömmlicher Weise mit monoklonalen Mausantikörpern gegen rekombiniertes Interferon αA sensibilisiert. Die Kugeln werden aus der Pufferlösung entnommen, in einem groben Sieb abgetropft und während 10 Minuten bei Raumtemperatur in einer wässrigen Lösung enthaltend 50 g/l Kaliumgalacturonat suspendiert. Anschliessend werden die Kugeln mit einem Sieb aus dieser Lösung entnommen, abgetropft und während 24 Stunden bei 37°C getrocknet.

## Beispiel 1b

Polystyrolkugeln (Durchmesser 6,35 mm) werden in Pufferlösung in herkömmlicher Weise mit monoklonalen Mausantikörpern gegen rekombiniertes Interferon αA sensibilisiert. Die Kugeln werden aus der Pufferlösung entnommen, in einem groben Sieb abgetropft und während 10 Minuten bei Raumtemperatur in einer wässrigen Lösung enthaltend 50 g/l Kaliummannonat suspendiert. Anschliessend werden die Kugeln mit einem Sieb aus dieser Lösung entnommen, abgetropft und während 24 Stunden bei 37°C getrocknet.

## Beispiel 2

Polystyrolkugeln (Durchmesser 6,35 mm) werden in Pufferlösung in herkömmlicher Weise mit monoklonalen Mausantikörpern gegen CEA sensibilisiert. Die Kugeln werden aus der Pufferlösung entnommen, in einem groben Sieb abgetropft und während 10 Minuten bei Raumtemperatur in eine wässrige Lösung enthaltend 100 g/l Natriumglucuronat suspendiert. Anschliessend werden die Kugeln mit einem Sieb aus der Lösung entnommen, abgetropft und während 1 Stunde an Rotationsverdampfer unter Vakuum bei 37°C getrocknet.

Ein Teil der Kugeln wird bei Raumtemperatur während 1 Monat gelagert während ein anderer Teil während 1 Monat bei 37°C gelagert wird.

Alle Kugeln zeigen nach der Lagerung nur äusserst geringfügige Verluste an immunologischer Aktivität. Die Kugeln bleiben nach Kontakt mit Luftfeuchtigkeit trocken.

## Beispiel 3

Polystyrolkugeln (Durchmesser 6,35 mm) werden in Pufferlösung in herkömmlicher Weise mit monoklonalen Mausantikörpern gegen HCG sensibilisiert. Die Kugeln werden aus der Pufferlösung entnommen, in einem groben Sieb abgetropft und während 10 Minuten bei Raumtemperatur in einer wässrigen Lösung enthaltend 100 g/l Natriumglucuronat suspendiert. Anschliessend werden die Kugeln mit einem Sieb aus der Lösung entnommen, abgetropft und während 1 Stunde am Rotationsverdampfer unter Vakuum bei 37°C getrocknet.

Ein Teil der Kugeln wird während 1 Monat bei Raumtemperatur und ein anderer Teil der Kugeln während 1 Monat bei 37°C gelagert.

Alle Kugeln zeigen nach der Lagerung nur äusserst geringfügige Verluste an immunologischer Aktivität. Die Kugeln bleiben nach Kontakt mit Luftfeuchtigkeit trocken.

## Beispiel 4

a) Polystyrolkugeln (Durchmesser 6,35 mm) werden in Pufferlösung in herkömmlicher Weise mit monoklonalen Mausantikörpern gegen Hepatitis B-Oberflächenantigen sensibilisiert. Die Kugeln werden aus der Pufferlösung entnommen, in einem groben Sieb abgetropft und während 10 Minuten bei Raumtemperatur in eine wässrige Lösung enthaltend 100 g/l Natriumglucuronat suspendiert. Anschliessend werden die Kugeln mit einem Sieb aus der Lösung entnommen, abgetropft und während 1 Stunde im Rotationsverdampfer unter Vakuum bei 37°C getrocknet.

Ein Teil der Kugeln wird während 1 Monats bei Raumtemperatur und ein Teil der Kugeln während 1 Monats bei 37°C gelagert.

Alle Kugeln zeigen nach der Lagerung nur einen äusserst geringfügigen Verlust an immunologischer Aktivität. Die Kugeln bleiben nach Kontakt mit Luftfeuchtigkeit trocken.

Ein Teil der Kugeln wurde während 7 Monaten bei Raumtemperatur gelagert, wonach sie immer noch 70% der immunologischen Aktivität aufwiesen.

b) Polystyrolkugeln (Durchmesser 6,35 mm) werden in Pufferlösung in herkömmlicher Weise mit monoklonalen Mausantikörpern gegen Hepatitis B-Oberflächenantigen sensibilisiert. Die Kugeln werden aus der Pufferlösung entnommen, in einem groben Sieb abgetropft und während 10 Minuten bei Raumtemperatur in eine wässrige Lösung enthaltend 100 g/l Natriumgluconat suspendiert. Anschliessend werden die Kugeln mit einem Sieb aus der Lösung entnommen, abgetropft und während 10 Minuten im Wirbelschichtgranulator bei 37°C getrocknet.

Ein Teil der Kugeln wird während 1 Monats bei Raumtemperatur und ein Teil der Kugeln während 1 Monats bei 35°C gelagert.

Alle Kugeln zeigen nach der Lagerung nur wenig Veränderung an immunologischer Aktivität. Die Kugeln bleiben nach Kontakt mit Luftfeuchtigkeit trocken.

## Beispiel 5

Polystyrolkugeln (Durchmesser 6,35 mm) werden in Pufferlösung in herkömmlicher Weise mit monoklonalen Mausantikörpern gegen humanes IgE sensibilisiert. Die Kugeln werden aus der Pufferlösung entnommen, in einem groben Sieb abgetropft und während 10 Minuten bei Raumtemperatur in einer wässrigen Lösung enthaltend 50 g/l Natriumglucuronat suspendiert. Anschliessend werden die Kugeln mit einem Sieb aus der Lösung entnommen, abgetropft und während 24 Stunden bei 37°C getrocknet.

Ein Teil der Kugeln wird während 1 Monat bei 2-8°C, ein anderer Teil bei Raumtemperatur und ein dritter Teil bei 37°C gelagert.

Alle Kugeln zeigen nach der Lagerung nur einen äusserst geringfügigen Verlust an immunologischer Aktivität. Die Kugeln bleiben nach Kontakt mit Luftfeuchtigkeit trocken.

## Beispiel 6

### Bestimmung von Inteferon in Patientenserum (Enzym-Immunverfahren)

In die entsprechende Anzahl Teströhrchen (10 x 75 mm) werden je 0,05 ml Testlösung (0,1 Mol/l Natriumphosphat, pH 6,5 mit 10 g/l BSA und 0,5 µg/ml monoklonales Maus-Anti-Interferon-Peroxidase-Konjugat in Interferon-freiem Humanserum) pipettiert, 0,2 ml der zu analysierenden Patientenplasmen respektive der Interferon-Standards (0 U/ml, 12,5 U/ml, 25 U/ml und 50 U/ml Interferon) und des Interferon-Kontrollserums (z.B. 30 U/ml Interferon) zugemischt, je eine mit monoklonalen Mausantikörpern gegen rekombiniertes Interferon αA beschichtete und gemäss Beispiel 1 mit Natriumglucuronat überzogene Polystyrolkugel (∅ = 6,5 mm) zugefügt und bei Raumtemperatur (18-26°C) während 16 Stunden inkubiert. Anschliessend werden die Polystyrolkugeln dreimal mit je 2 bis 5 ml destilliertem Wasser gewaschen, in je 0,5 ml Substratpuffer für die Aktivitätsbestimmung der Peroxidase (0,1 Mol/l Kaliumcitratpuffer vom pH 5,0 mit 6 mMol/l $H_2O_2$ und 20 mMol/l o-Phenylendiamin) transferiert und während 30 Minuten bei Raumtemperatur (18-26°C) inkubiert. Zum Abstoppen der peroxidatischen Aktivität sowie zur Intensivierung der Lichtabsorption werden 0,5 ml 4 N HCl zugemischt und innerhalb von 30 Minuten die Extinktion bei der Wellenlänge 492 nm photometrisch gemessen. Die $\Delta E$ Werte sind Mittel aus Doppelbestimmungen. In Tabelle I sind die Werte einer Interferon-Bestimmung aufgeführt und mit den Werten verglichen, wie sie mit den Interferon-Standards erhalten wurden.

Tabelle I

| Probenmaterial | $\Delta E_{492\ nm/RT/30\ min}$ | |
|---|---|---|
| Interferon-Standard | | |
| 0 U/ml Interferon | 0.0675 | |
| 12,5 U/ml " | 0.24 | |
| 25 U/ml " | 0.427 | |
| 50 U/ml " | 0.782 | |
| Kontrollserum (30 U/ml Interferon) | 0.497 | |
| Patientenplasmen | | |
| No. 8327 | 0.060 | 0 U/ml interferon |
| No. 8328 | 0.060 | 0 U/ml " |
| No. 8336 | 0.205 | 9 U/ml " |
| No. 8338 | 0.062 | 0 U/ml " |
| No. 8339 | 0.145 | 5 U/ml " |
| No. 8363 | 0.058 | 0 U/ml " |
| No. 8341 | 0.103 | 2 U/ml " |
| No. 8344 | 0.302 | 16 U/ml " |

Beispiel 7

Quantitative Bestimmung von CEA in Patientenplasmen

In die erforderliche Anzahl Teströhrchen (10 x 75 mm) werden je 0,2 ml Testlösung (0,2 Mol/l $NaH_2PO_4/Na_2HPO_4$, pH 6,5 mit 2 g/l Rinderserumalbumin, 20% normales Ziegenserum und 0,2 µg/ml Ziegen-Anti-CEA-Peroxidase-Konjugat) pipettiert, 0,050 ml der zu analysierenden Patientenplasmen resp. der CEA-Standards (0 ng/ml CEA, 2,5 ng/ml CEA, 10 ng/ml CEA und 20 ng/ml CEA) und des CEA-Kontrollserums (5,0 ng/ml CEA ± 1,0 ng/ml) zugemischt, je eine mit monoklonalem Maus-Anti-CEA-sensibilisierte und gemäss Beispiel 2 mit Natriumglucuronat überzogene Polystyrolkugel ($\emptyset$ = 6,5 mm) zugefügt und bei 37°C während 16 Stunden inkubiert. Anschliessend werden die Polystyrolkugeln dreimal mit je 2-5 ml dest. Wasser gewaschen, in je 0,5 ml Substratpuffer für die Aktivitätsbestimmung der Peroxidase (0,1 Mol/l Kaliumcitratpuffer vom pH 5,0 mit 6 mMol/l $H_2O_2$ und 20 mMol/l o-Phenylendiamin) transferiert und während 30 Minuten bei Raumtemperatur (22°C) inkubiert. Zum Abstoppen der peroxidatischen Aktivität sowie zur Intensivierung der Farbintensität werden 2,0 ml 1N HCl zugemischt und innerhalb von 30 Minuten die Extinktion bei der Wellenlänge 492 nm photometrisch gemessen. Die $\Delta E$ Werte sind Mittel aus Doppelbestimmungen. In Tabelle II sind die Werte einer CEA-Bestimmung aufgeführt und mit den Werten verglichen, wie sie mit dem Radioimmunoassay von ROCHE erhalten werden.

0133976

## Tabelle II

| Probenmaterial | $\Delta E_{492}$ nm/RT/30 min. | |
|---|---|---|
| **CEA-Standard** | | |
| 0    ng/ml CEA | **0.103** | |
| 2,5 ng/ml CEA | **0.330** | |
| 10,0 ng/ml CEA | **0.978** | |
| 20,0 ng/ml CEA | **.1.859** | |
| **CEA-Kontrollserum** | | |
| 5,0 ng/ml CEA | **0.540** | |

| Patienten-plasmen | ROCHE-RIA-Test | erfindungsgemässes Verfahren | |
|---|---|---|---|
| | | $\Delta E_{492}$ nm/RT/30 min. | CEA ng/ml |
| No. 7212 | **0.6 ng/ml** | 0.203 | **1.2** |
| No. 7188 | **2.2 ng/ml** | 0.185 | **1.0** |
| No. 7220 | **1.2 ng/ml** | 0.227 | **1.4** |
| No. 7218 | **1.2 ng/ml** | 0.215 | **1.3** |
| No. 7234 | **2.5 ng/ml** | 0.350 | **2.7** |
| No. 7249 | **2.4 ng/ml** | 0.285 | **2.0** |
| No. 7203 | **2.3 ng/ml** | 0.285 | **2.0** |
| No. 7223 | **3.0 ng/ml** | 0.399 | **3.3** |
| No. 7247 | **3.1 ng/ml** | 0.355 | **2.8** |
| No. 7258 | **4.6 ng/ml** | 0.510 | **4.3** |
| No. 7215 | **4.9 ng/ml** | 0.585 | **5.5** |
| No. 7219 | **5.0 ng/ml** | 0.610 | **5.9** |
| No. 8180 | **8.6 ng/ml** | 0.850 | **8.5** |
| No. 7248 | **11.2 ng/ml** | 1.045 | **10.7** |
| No. 7262 | **14.2 ng/ml** | 1.360 | **14.3** |
| No. 7201 | **15.6 ng/ml** | 1.445 | **15.3** |

Werte unter 2,5 ng/ml CEA liegen im Normalbereich, während Werte über 2,5 ng/ml im pathologischen Bereich liegen.

## Beispiel 8

### Quantitative Bestimmung von HCG im Serum

In die erforderliche Anzahl Teströhrchen (10 x 75 mm) werden je 0,2 ml Testlösung (0,1 Mol/l $NaH_2PO_4$/$Na_2HPO_4$, pH 7,0 mit 2 g/l Rinderserumalbumin, und 1,0 µg/ml monoklonale Maus-Anti-HCG-Peroxidase-Konjugat pipettiert, 0,050 ml der zu analysierenden Patientenplasmen resp. der HCG-Standards (0, 25, 50, 100 und 200 IU/l HCG) zugemischt, je eine mit monoklonalem Maus-Anti-HCG-sensibilisierte und gemäss Beispiel 3 mit Natriumglucuronat überzogene Polystyrolkugel (Ø = 6,5 mm) zugefügt und bei 37°C während 2 Stunden inkubiert. Anschliessend werden die Polystyrolkugeln dreimal mit je 2-5 ml dest. Wasser gewaschen, in je 0,5 ml Substratpuffer für die Aktivitätsbestimmung der Peroxidase (0,1 Mol/l Kaliumcitratpuffer vom pH 5,0 mit 6 mMol/l $H_2O_2$ und 20 mMol/l o-Phenylendiamin) transferiert und während 30 Minuten bei Raumtemperatur (18-26°C) inkubiert. Zum Abstoppen der peroxidatischen Aktivität sowie zur Intensivierung der Farbintensität werden 1,0 ml 2N HCl zugemischt und innerhalb von 30 Minuten die Extinktion bei der Wellenlänge 492 nm photometrisch gemessen. Die $\triangle$E Werte sind Mittel aus Doppelbestimmungen. In Tabelle III sind die Werte von HCG-Bestimmungen in Serum und in Tabelle IV von Patientenseren aufgeführt.

### HCG-Bestimmung in Humanserum

#### Tabelle III

1. Standardkurven

| IU/l HCG | 1) Serum | $\triangle E_{492}$ nm/30 Min./RT | | |
| --- | --- | --- | --- | --- |
| | | 2) Plasma | 3) Urin | 4) Puffer |
| 0 | 0,038 | 0,054 | 0,158 | 0,180 |
| 25 | 0,226 | - | - | - |
| 50 | 0,475 | 0,544 | 0,557 | 0,716 |
| 100 | 0,905 | 0,955 | 0,970 | 1,40 |
| 200 | 1,75 | 1,55 | 1,90 | 2,44 |

Die HCG-Werte der unten angeführten Beispiele von Patientenseren wurden aus der Standardkurve 1) abgelesen. Die Standardkurven 2), 3) und 4) wurden an einem andern Tag mit neuen HCG Standards ermittelt.

Tabelle IV

2.   Patientenseren

| Serum | $\Delta E_{492}$/30 Min./RT gemessen | IU/l HCG aus Kurve 1) |
|---|---|---|
| Pool 091080 | 0,041 | 0 |
| Nr. 2801 | 0,025 | 0 |
| Nr. 3881 | 0,450 | 47 |
| Nr. 2673 | 1,13 | 127 |
| Nr. 1167 | 2,12 (1:2) | 470 |
| Nr. 4891 | 0,975 (1:50) | 5'450 |
| Nr. 3240 | 1,06 (1:20) | 2'280 |
| Nr. 4418 | 1,475 (1:1000) | 167'000 |

Beispiel 9

Qualitative Bestimmung von HBsAg in Humanserum oder -plasma

a)   In die erforderliche Anzahl Teströhrchen (10 x 60 mm bei denen mit 200 µl die Kugel bedeckt ist) werden je 0,1 ml der zu analysierenden Probe respektive der negativen oder positiven (entsprechend ca. 20 ± 5 ng/ml HBsAg) Kontrolle pipettiert, je 0,1 ml Testlösung (0,1 Mol/l Tris-Acetat pH 7,3, mit 0,5 g/l Thimerosal, 0,4 g/l 4-Amino-antipyrin, 0,2 g/l Tween-20, 40'000 U USP/l Natrium-Heparinat, 20% V/V foetales Kälberserum und 0,1-0,4 mg/l Ziegen-anti-HBs-Peroxidase-Konjugat) zugemischt, je eine mit monoklonalem Maus-anti-HBs sensibilisierte und gemäss Beispiel 4 mit Natriumglucuronat überzogene Polystyrol-kugel (∅ = 6,35 mm) zugefügt und bei 45°C im Wasserbad während 2 Stunden inkubiert.

Anschliessend werden die Polystyrolkugeln fünfmal mit je 3-5 ml Waschlösung (ca. 5,0 mMol/l $Na_2HPO_4$, 1,5 mMol/l $KH_2PO_4$, ca. 130 mMol/l NaCl und 0,02 g/l Tween-20) gewaschen, je 0,25 ml Substratpuffer für die Aktivitätsbestimmung der Peroxidase (0,1 Mol/l Kaliumcitratpuffer vom pH 5,0 mit 6 mMol/l $H_2O_2$ und 20 mMol/l o-Phenylendiamin) zugegeben und während 30 Minuten bei Raumtemperatur (22°C) inkubiert. Zum Abstoppen der peroxidatischen Aktivität sowie zur Intensivierung der Farbintensität werden 1,0 ml 1N $H_2SO_4$ zugemischt und innerhalb von 30 Minuten die Extinktion bei der Wellenlänge 490-492 nm photometrisch gemessen. Die $\Delta E$ Werte sind Mittel aus Doppelbestimmungen.

In Tabelle V sind die $\Delta E$-Werte einer Serie von HBsAg-Bestimmungen aufgeführt.

## Tabelle V

| Probe | $\Delta E$ | Probe | $\Delta E$ |
|---|---|---|---|
| 1 | > 1,500 | 16 | 0,111 |
| 2 | > 1,500 | 17 | 0,099 |
| 3 | > 1,500 | 18 | 0,024 |
| 4 | > 1,500 | 19 | 0,028 |
| 5 | 0,869 | 20 | 0,024 |
| 6 | 0,499 | 21 | 0,032 |
| 7 | 0,138 | 22 | 0,023 |
| 8 | 0,374 | | |
| 9 | 0,198 | Kontrollen | |
| 10 | 0,118 | Positive | 0,826 |
| 11 | 0,371 | | 0,879 |
| 12 | 0,398 | | 0,849 |
| 13 | 0,370 | Negative | 0,040 |
| 14 | 0,226 | | 0,033 |
| 15 | 0,246 | | 0,031 |

b)    In die erforderliche Anzahl Teströhrchen (10 x 60 mm bei denen mit 200 µl die Kugel bedeckt ist) werden je 0,1 ml der zu analysierenden Probe respektive der negativen oder positiven (entsprechend ca. 20 $\pm$ 5 ng/ml HBsAg) Kontrolle pipettiert, je 0,1 ml Testlösung (0,1 Mol/l Tris-Acetat pH 7,3, mit 0,5 g/l Thimerosal, 0,4 g/l 4-Aminpantipyrin, 0,2 g/l Tween-20, 40'000 U USP/l Natrium-Heparinat, 20% V/V foetales Kälberserum und 0,1-0,4 mg/l Ziegen-anti-HBs-Peroxidase-Konjugat) zugemischt, je eine mit monoklonalem Maus-anti-HBs sensibilisierte und gemäss Beispiel 4 b) mit Natriumgluconat überzogene Polystyrolkugel ($\emptyset$ = 6,35 mm) zugefügt und bei 45°C im Wasserbad während 2 Stunden inkubiert.

Anschliessend werden die Polystyrolkugeln fünfmal mit je 3-5 ml Waschlösung (ca. 5,0 mMol/l $Na_2HPO_4$, ca. 1,5 mMol $KH_2PO_4$, ca. 130 mMol/l NaCl und 0,02 g/l Tween-20) gewaschen, je 0,25 ml Substratpuffer für die Aktivitätsbestimmung der Peroxidase (0,1 Mol/l Kaliumcitratpuffer vom pH 5,0 mit 6 mMol/l $H_2O_2$ und 20 mMol/l o-Phenylendiamin) zugegeben und während 30 Minuten bei Raumtemperatur (22°C) inkubiert. Zum Abstoppen der peroxidatischen Aktivität sowie zur Intensivierung der Farbintensität werden 1,0 ml 1N $H_2SO_4$ zugemischt und innerhalb von 30 Minuten die Extinktion bei der Wellenlänge 490-492 nm photometrisch gemessen. Die $\Delta$E Werte sind Mittel aus Doppelbestimmungen.

In Tabelle VI sind die $\Delta$E -Werte einer Serie von HBsAg-Bestimmungen aufgeführt, erhalten mit Kugeln zu Beginn (AA) und nach 1 Monat Lagerung bei 24-26°C respektive 35°C.

## TabelleVI

| | $\Delta E_{E_{492}nm}$ AA | $\Delta E_{E_{492}nm}$ 1 Monat 24-26°C | $\Delta E_{E_{492}nm}$ 1 Monat 35°C |
|---|---|---|---|
| Probe 1 | 0,190 | 0,153 | 0,112 |
| Probe 2 | 0,094 | 0,109 | 0,084 |
| Probe 3 | 0,100 | 0,085 | 0,083 |
| Probe 4 | 0,074 | 0,070 | 0,055 |
| Probe 5 | 0,789 | 0,790 | 0,881 |
| Probe 6 | 0,927 | 0,896 | 1,013 |
| Probe 7 | 0,949 | 0,902 | 1,098 |
| Probe 8 | 1,066 | 1,072 | 1,203 |
| Probe 9 | 0,577 | 0,696 | 0,663 |

## Beispiel 10

### Quantitative Bestimmung von Human IgE in Patientenseren oder -plasmen

In die erforderliche Anzahl Teströhrchen (10 x 75 mm) werden je 0,2 ml Testlösung (0,1 Mol/1 TRIS/Acetat, pH 7,25 mit 200 ml/1 hitzebehandeltem (56°/30 Min.) foetalem Kälberserum, 0,25 g/1 Thimerosal und 0,2 mg/1 monoklonales (Maus) Anti-Human IgE-Peroxidase-Konjugat) pipettiert, 0,05 ml der zu untersuchenden Serum- oder Plasmaproben resp. der Human IgE-Standards mit 400 U/ml IgE, 200 U/ml IgE, 100 U/ml IgE, 50 U/ml IgE und 0 U/ml IgE zugemischt, je eine mit monoklonalem (Maus) Anti-Human IgE-sensibilisierte und gemäss Beispiel 5 mit Natriumglucuronat überzogene Polystyrolkugel (Ø = 6,35 mm) zugefügt und bei 18-26°C während 2 Stunden inkubiert. Anschliessend werden die Polystyrolkugeln dreimal mit je 3-5 ml entionisiertem Wasser gewaschen, in je 0,5 ml o-Phenylendiamin-Substratpuffer für die Aktivitätsbestimmung der Peroxidase (0,1 Mol/1 Kaliumcitrat, pH 5,0 mit 6 mMol/1 $H_2O_2$ und 20 mMol/1 o-Phenylendiamin) während 30 Minuten bei 18-26°C inkubiert. Zum Abstoppen der peroxidatischen Aktivität sowie zur Intensivierung und Stabilisierung der Farbintensität werden 2,0 ml 0,5 Mol/1 $H_2SO_4$ - 1 g/1 $Na_2S_2O_5$ zugemischt und die Extinktion bei der Wellenlänge 492 nm photometrisch gemessen. Die $\Delta E$ Werte sind Mittel aus Doppelbestimmungen. In Tabelle VII sind die Werte einer Human IgE-Bestimmung aufgeführt.

## Tabelle VII

| Probenmaterial | $\Delta E_{492\ nm/}$ 30 min./22$^\circ$C | |
|---|---|---|
| Human IgE-Standard | | |
| 0 U/ml | 0.175 | |
| 50 U/ml | 0.570 | |
| 100 U/ml | 0.795 | |
| 200 U/ml | 1.010 | |
| 400 U/ml | 1.190 | |
| Serumproben | | Human IgE-Gehalt |
| No. 129 | 1.133 | 330 U/ml |
| No. 139 | 1.175 | 385 U/ml |
| No. 140 | 1.220 | 468 U/ml |
| No. 141 | 1.095 | 280 U/ml |
| No. 146 | 1.115 | 300 U/ml |
| No. 154 | 0.995 | 190 U/ml |
| No. 163 | 1.210 | 440 U/ml |
| No. 173 | 1.160 | 360 U/ml |
| No. 174 | 1.088 | 270 U/ml |
| No. 178 | 1.068 | 250 U/ml |

- 20 -    0133976

### Patentansprüche

1. Reagens zur Durchführung von Immunverfahren, enthaltend einen mit einem immunologisch aktiven Material beschichteten festen Träger, dadurch gekennzeichnet, dass der beschichtete Träger mit einem Salz einer Zuckersäure überzogen ist.

2. Reagens nach Anspruch 1, dadurch gekennzeichnet, dass das Immunverfahren ein Enzym-Immun-Verfahren ist.

3. Reagens nach Anspruch 1 oder 2, dadurch gekennzeichnet, dass das immunologisch aktive Material ein Antigen ist.

4. Reagens nach Anspruch 1 oder 2, dadurch gekennzeichnet, dass das immunologisch aktive Material ein Antikörper ist.

5. Reagens nach Anspruch 1 oder 2, dadurch gekennzeichnet, dass das immunologisch aktive Material ein Hapten ist.

6. Reagens nach Anspruch 4, dadurch gekennzeichnet, dass der Antikörper ein Antikörper gegen Hepatitis B Oberflächenantigen ist.

7. Reagens nach Anspruch 6, dadurch gekennzeichnet, dass der Antikörper ein monoklonaler Maus-Antikörper ist.

8. Reagens nach Anspruch 4, dadurch gekennzeichnet, dass der Antikörper ein CEA-Antikörper ist.

9. Reagens nach Anspruch 8, dadurch gekennzeichnet, dass der CEA-Antikörper ein monoklonaler Maus-Antikörper ist.

10. Reagens nach Anspruch 4, dadurch gekennzeichnet, dass der Antikörper ein HCG Antikörper ist.

11. Reagens nach Anspruch 10, dadurch gekennzeichnet, dass der Antikörper ein monoklonaler Maus-Antikörper ist.

12. Reagens nach Anspruch 4, dadurch gekennzeichnet, dass der Antikörper ein Interferon-Antikörper ist.

13. Reagens nach Anspruch 12, dadurch gekennzeichnet, dass der Antikörper ein Antikörper gegen rekombiniertes Interferon αA ist.

14. Reagens nach Anspruch 13, dadurch gekennzeichnet, dass der Antikörper ein monoklonaler Maus-Antikörper ist.

15. Reagens nach Anspruch 4, dadurch gekennzeichnet, dass der Antikörper gegen humanes IgE gerichtet ist.

16. Reagens nach Anspruch 15, dadurch gekennzeichnet, dass der Antikörper ein monoklonaler Maus-Antikörper ist.

17. Reagens nach einem der Ansprüche 1-16, dadurch gekennzeichnet, dass der Träger eine oberflächenbehandelte Polystyrolkugel ist.

18. Reagens nach einem der Ansprüche 1-17, dadurch gekennzeichnet, dass das Salz einer Zuckersäure ein Glucuronsäuresalz ist.

19. Reagens nach einem der Ansprüche 1-17, dadurch gekennzeichnet, dass das Salz einer Zuckersäure ein Mannonsäuresalz ist.

20. Reagens nach einem der Ansprüche 1-17, dadurch gekennzeichnet, dass das Salz einer Zuckersäure ein Galacturonsäuresalz ist.

21. Reagens nach einem der Ansprüche 1-17, dadurch gekennzeichnet, dass das Salz einer Zuckersäure ein Gluconsäuresalz ist.

22. Reagens nach einem der Ansprüche 18-21, dadurch gekennzeichnet, dass die Salze Alkali- oder Ammoniumsalze sind.

23. Reagens nach Anspruch 22, dadurch gekennzeichnet, dass die Salze die Natriumsalze sind.

24. Reagens nach Anspruch 23, dadurch gekennzeichnet, dass das Salz Natriumglucuronat ist.

25. Reagens nach Anspruch 23, dadurch gekennzeichnet, dass das Salz Natriumgluconat ist.

***